# EUROPEAN PATENT APPLICATION

(11) **EP 1 681 069 A1**
(43) Date of publication of application: **19.07.2006**
(21) Application number: 04793098.7
(22) Date of filing: 28.10.2004
(51) Int. Cl.: A61M 5/142

(54) **MEDICINAL LIQUID INFUSION APPARATUS**

(30) Priority: 29.10.2003 JP 2003368859
(71) Applicant: Nemoto Kyorindo Co., Ltd., Tokyo 113-0033 (JP)
(72) Inventor: NEMOTO, Shigeru, c/o Nemoto Kyorindo Co., Ltd., Tokyo 1130033 (JP); KANETAKA, Toshio, c/o Nemoto Kyorindo Co., Ltd., Tokyo 1130033 (JP)
(74) Representative: Nargolwalla, Cyra
(86) International application number: PCT/JP2004/015990
(87) International publication number: WO 2005/039675

(57) **Abstract**

A chemical liquid injector (100), wherein various data on normal infusion is inputted from and displayed on a large and high-resolution main touch-panel (104) mounted on an infusion control unit (101) so that complicated input operation and data display for normal infusion can be accurately performed. Since various data on test infusion can be inputted and displayed on a small and low-resolution sub touch-panel (118) mounted on an infusion head (110), input operation and data check for test infusion can be easily performed while one worker monitors an extension tube (230) near the infusion head (110). Thus, the chemical liquid injenctor (100) enabling the test infusion to be easily and securely performed by one worker can be provided.

## Description

### Technical Field

The present invention relates to a chemical liquid (medical liquid) injector for injecting a liquid into a patient, and more particularly to a chemical liquid injector for injecting a contrast media into a patient who is to be imaged by an imaging diagnostic apparatus such as a CT (Computed Tomography) scanner, an MRI (Magnetic Resonance Imaging) apparatus, and a PET (Positron Emission Tomography) apparatus.

### Background Art

Presently available imaging diagnostic apparatuses for capturing diagnostic images of patients include CT scanners, MRI apparatuses, PET apparatuses, ultrasonic diagnostic apparatuses, CT angiography apparatuses, and MR angiography apparatuses. When such an apparatus is used, a liquid such as a contrast media or physiological saline may be injected into the patient. Chemical liquid injectors for automatically performing the injection have been put into practical use.

Such a chemical liquid injector has a drive motor, a slider mechanism and the like, and employs a liquid syringe which is removably mounted, for example. The liquid syringe comprises a cylinder member and a piston member slidably inserted in the cylinder member. The cylinder member is filled with a liquid such as a contrast media or physiological saline.

The liquid syringe is connected to the patient through an extension tube and set on a piston driving mechanism. The piston driving mechanism individually holds the piston member and the cylinder member and moves them relatively to inject the liquid, typically the contrast media, from the liquid syringe into the patient.

An operator determines the rate at which the contrast media is injected and the total volume of the contrast media to be injected, in view of various conditions, and then enters numerical data representing the rate and total volume into the chemical liquid injector. The chemical liquid injector injects the contrast media into the patient at the rate and in the quantity represented by the entered numerical data. The injected contrast media changes the image contrast of the patient, allowing the imaging diagnostic apparatus to capture a good diagnostic image of the patient.

Some chemical liquid injectors are capable of injecting the physiological saline as well as the contrast media into the patient. In such a chemical liquid injector, a contrast media syringe filled with the contrast media and a physiological saline syringe filled with the physiological saline are mounted side by side as liquid syringes and are typically connected to the patient through a single bifurcated extension tube.

The operator enters, as desired, an instruction to inject the physiological saline following the completion of the injection of the contrast media, together with data representing the injection rate and total volume of the physiological saline, into the chemical liquid injector. Based on the entered data, the chemical liquid injector first injects the contrast media into the patient and then automatically injects the physiological saline. The subsequently injected physiological saline, which pushs the previously injected contrast media, functions to reduce the consumption of the contrast media, and also to reduce artifacts in the captured image.

For injecting the liquid from the liquid syringe into the patient through the extension tube in the chemical liquid injector as described above, test injection is typically performed first to check the connection of the extension tube. In the test injection, a small amount of liquid is injected at a higher pressure than in normal injection, and the pressure of the injected liquid is monitored.

For example, if the detected pressure is below a predetermined permissible range, it shows that the liquid is leaked from the extension tube. On the other hand, if the detected pressure is above the predetermined permissible range, it shows that the liquid is not appropriately injected into the blood vessel of the patient from the connection tube.

The test injection for checking the connection of the extension tube is performed naturally with the contrast media in the chemical liquid injector on which only the contrast media syringe is mounted, while it is generally performed with the physiological saline in the chemical liquid injector on which both of the contrast media syringe and the physiological saline syringe are mounted, in light of the cost involved.

Test injection for measuring a time period necessary for the contrast media to reach an affected area of a patient is also performed as the test injection prior to the normal injection. In the test injection, the contrast media is injected at the same rate as in the normal injection to measure the time period from the start of the injection to the arrival of the contrast media at the affected area.

Currently widespread chemical liquid injectors have an injection head for mounting the liquid syringe and an injection control unit for data display and input actions, as separate components. Thus, the injection head can be disposed near the patient as required, while the injection control unit can be placed near an imaging control unit of the imaging diagnostic apparatus or the like.

Especially when the imaging diagnostic apparatus is an MRI apparatus, the injection control unit containing a microcomputer cannot be placed near a diagnostic imaging unit of the MRI apparatus. The abovementioned structure is advantageous since the injection head and the injection control unit are formed as the separate components and disposed away from each other.

Chemical liquid injectors of the type described above have been devised and applied for patent by the applicant of the present application and the like (see, for example, patent documents 1, 2 below).

### List of References

Patent document 1: Japanese laid-open patent publication No. 2002-11096;
Patent document 2: Japanese laid-open patent publication No. 2002-102343.

In the chemical liquid injectors described above, since the operation of liquid injection is controlled in response to input actions made by the operator, the test injection and normal injection can be performed appropriately. The normal injection requires input of operation conditions in liquid injection in view of various factors such as the weight and affected area of a patient. In the test injection, it is only necessary to perform standard injection operation regardless of the weight and affected area of a patient.

In the conventional chemical liquid injectors, however, the test injection requires input of injection conditions as in the normal injection, and the input actions are cumbersome and may cause erroneous operation. To solve the problem, some chemical liquid injectors allow the test injection without having to perform the cumbersome input actions by registering control data dedicated to the test injection.

Since the current chemical liquid injectors include the injection head and the injection control unit which are separate components and are placed away from each other as described above, the operator makes input actions of various data and sees display output in the test injection on the injection control unit away from the injection head. However, in the test injection for checking the connection of the extension tube, the operator naturally needs to visually check the extension and the like near the injection head.

For example, when the test injection is performed in the current chemical liquid injector, one of two operators visually checks the extension tube and the like near the injection head, while the other should input the data and check the displayed data on the injection control unit, which means that much manpower is needed.

It is not impossible that a single operator performs the test injection by moving between the injection control unit and the injection head. However, this is burdensome for the operator and is likely to cause operation errors. It is also not impossible that the injection control unit is moved to near the injection head only in the test injection to allow a single operator to perform the test injection, but this also involves a heavy workload such as moving the unit and is not practical.

### Disclosure of the Invention

The present invention has been made in view of the abovementioned problems, and it is an object thereof to provide a chemical liquid injector which allows a single operator to perform test injection easily and reliably.

According to the present invention, the chemical liquid injection has an injection control unit and an injection head which are formed as separate components. The injection control unit is equipped with, at least, a computer unit, a main display panel, and a main operation panel. The injection head is equipped with, at least, a cylinder holding mechanism, a piston driving mechanism, a sub display panel, and a sub operation panel.

The cylinder holding member removably holds a cylinder member of a liquid syringe. The piston driving mechanism slides a piston member of the held liquid syringe. The computer unit controls the operation of the piston driving mechanism. The main display panel displays various data from the computer unit. The main operation panel accepts an input action of various data to the computer unit. The sub display panel is smaller than the main display panel and displays various data from the computer unit. The sub operation panel is smaller than the main operation panel and accepts an input action of various data to the computer unit.

In the chemical liquid injector according to the present invention, the liquid is injected from the liquid syringe into a patient through an extension tube in test injection before normal injection. The various data for the normal injection is input on the main operation panel and output as display on the main display panel. The various data for the test injection is input on the sub operation panel and output as display on the sub display panel.

The various means referred to in the present invention are not required to be individually independent entities, and may be arranged such that a plurality of means may be constructed as a single apparatus, a certain means may be part of another means, or part of a certain means and part of another means overlap each other.

### Effect of the Invention

In the chemical liquid injector according to the present invention, the various data for the normal injection is input on the main operation panel mounted on the injection control unit and output as display on the main display panel, so that complicated input actions and data display for the normal injection can be performed appropriately. In addition, the various data for the test injection is input on the sub operation panel mounted on the injection head and output as display on the sub display panel. Therefore, a single operator can easily make input actions and check data for the test injection while he or she visually checks the extension tube and the like near the injection head.

### Brief Description of the Drawings

Fig. 1 is a perspective view showing the exterior appearance of a chemical liquid injector of an imaging diagnostic system in an embodiment according to the present invention;
Fig. 2 is a perspective view showing how to mount a liquid syringe on an injection head of the chemical liquid injector;
Fig. 3 is a perspective view showing the exterior appearance of the imaging diagnostic system;
Fig. 4 is a cross-sectional view showing the internal structure of the injection head;
Fig. 5 is a block diagram showing the physical structure of the imaging diagnostic system;
Fig. 6 is a schematic diagram showing the logical structure of the imaging diagnostic system;
Fig. 7 is a schematic front view showing a display screen on a sub touch panel which serves both as a sub operation panel and a sub display panel;
Fig. 8 is a schematic front view showing schematic images of body sections and a condition screen with no data displayed on a main touch panel which serves both as a main operation panel and a main display panel;
Fig. 9 is a schematic front view showing the main touch panel on which a body section is selected;
Fig. 10 is a schematic front view showing the main touch panel on which a condition image for a selected region to be imaged is displayed;
Fig. 11 is a schematic front view showing the main touch panel on which the text data of the condition image is enlarged;
Fig. 12 is a flow chart showing a first part of a processing sequence of the chemical liquid injector;
Fig. 13 is a flow chart showing a subsequent part of the processing sequence;
Fig. 14 is a flow chart showing a subsequent part of the processing sequence;
Fig. 15 is a flow chart showing a last part of the processing sequence;
Fig. 16 is a flow chart showing the processing sequence of an MRI apparatus as an imaging diagnostic apparatus;
Fig. 17 is a schematic front view showing a display screen on a sub touch panel in a modification; and
Fig. 18 is a schematic front view showing a display screen on a sub touch panel in another modification.

### Description of Reference Numerals

- 100: Chemical Liquid Injector
- 101: Injection Control Unit
- 104: Main Touch Panel Serving As Main Operation Panel And Main Display Panel
- 110: Injection Head
- 116: Cylinder Holding Mechanism
- 117: Piston Driving Mechanism
- 118: Sub Touch Panel Serving As Sub Operation Panel And Sub Display Panel
- 120: Computer Unit
- 200: Liquid Syringe
- 210: Cylinder Member
- 220: Piston Member
- 300: MRI Apparatus Serving As Imaging Diagnostic Apparatus

### Best Mode for Carrving Out the Invention

### <Configuration of the Embodiment>

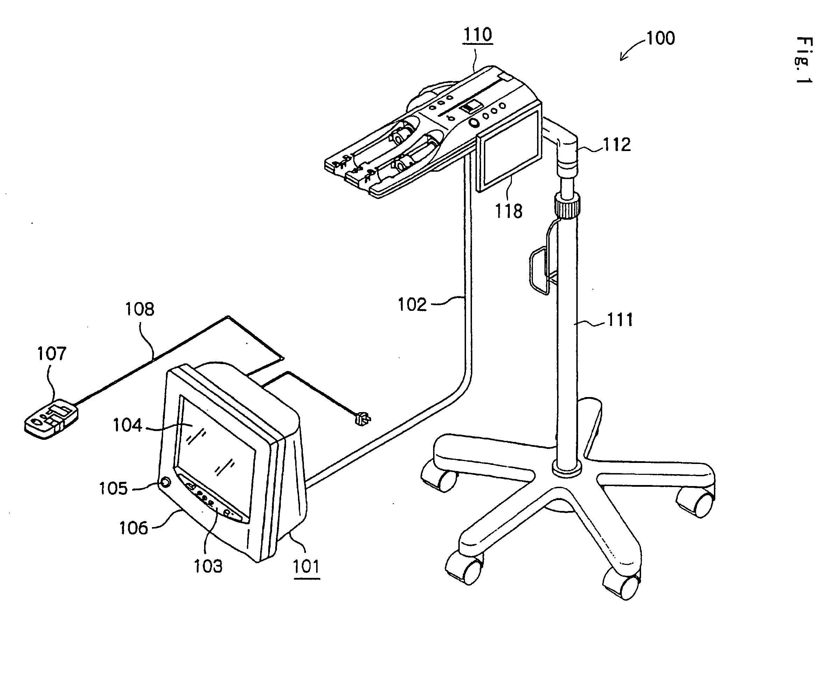

An embodiment of the present invention will hereinafter be described with reference to drawings. As shown in Figs. 1 to 4, chemical liquid injector system 1000 of the present embodiment according to the present invention comprises chemical liquid injector 100, liquid syringe 200, and MRI apparatus 300 which is a diagnostic imaging apparatus. The system is intended for injecting a contrast media or the like as a liquid into a patient (not shown), later described in detail.

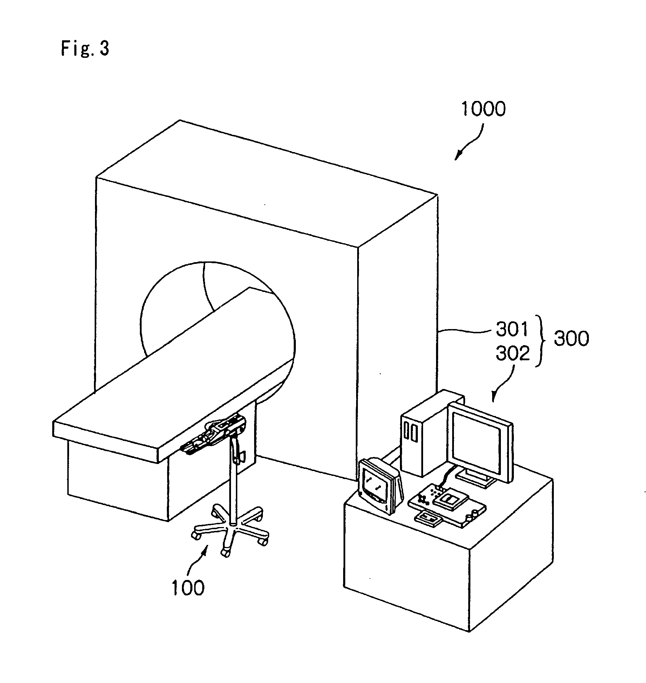

As shown in Fig. 3, MRI apparatus 300 includes diagnostic imaging unit 301 serving as a mechanism for performing imaging and imaging control unit 302 such that diagnostic imaging unit 301 and imaging control unit 302 are wired-connected through communication network 303. Diagnostic imaging unit 301 shoots a diagnostic image of a patient. Imaging control unit 302 controls the operation of diagnostic imaging unit 301.

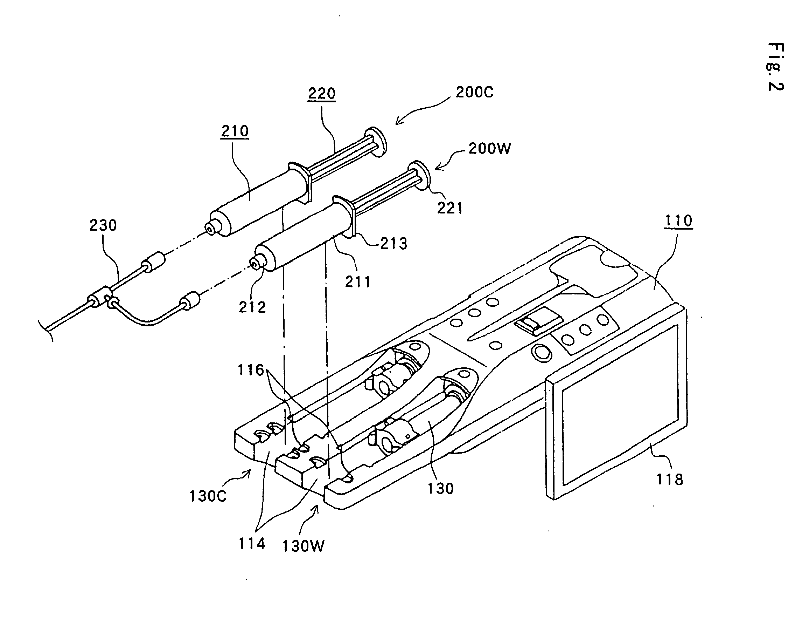

As shown in Fig. 2, liquid syringe 200 comprises cylinder member 210 and piston member 220 wherein piston member 220 is slidably inserted into cylinder member 210. Cylinder member 210 includes cylindrical hollow body 211 which has conduit tube 212 formed in the closed leading end.

The trailing end of body 211 of cylinder member 210 is opened and piston member 220 is inserted from the opening into the interior of body 211. Cylinder member 210 has cylinder flange 213 formed in the outer circumference of the trailing end, and piston member 220 has piston flange 221 formed in the outer circumference of the trailing end.

As shown in Fig. 1, chemical liquid injector 100 of the present embodiment 100 has injection control unit 101 and injection head 110 serving as an injector body, constructed as separate components which are wired-connected through communication cable 102. Injection head 110 drives liquid syringe 200 mounted thereon to inject a liquid therefrom into a patient. Injection control unit 101 controls the operation of injection head 110.

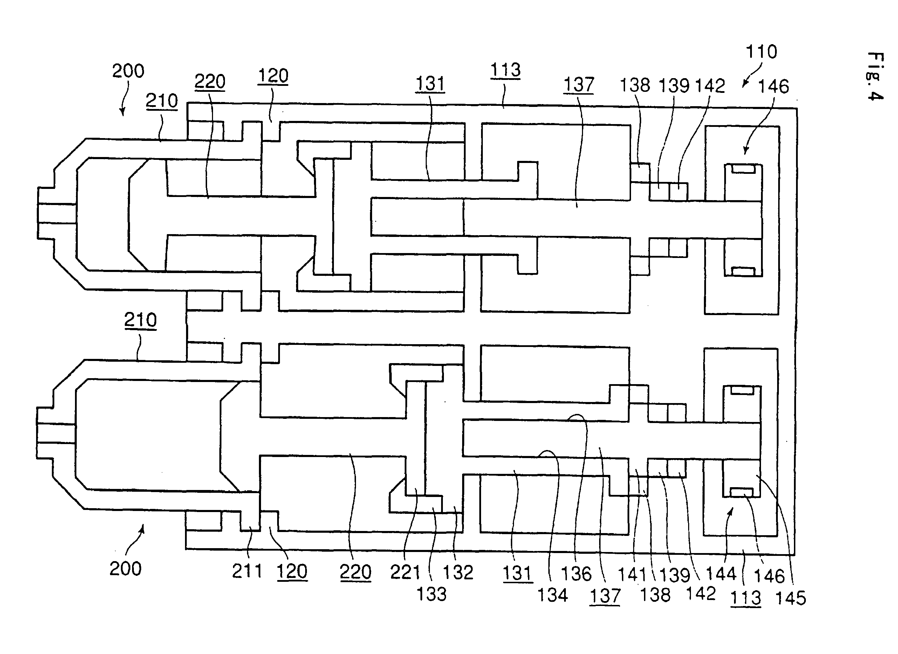

Thus, as shown in Fig. 4, injection control unit 101 contains computer unit 120 therein and is wire-connected to imaging control unit 302 of MRI apparatus 300 through communication network 304.

As shown in Fig. 1, injection control unit 101 has main operation panel 103, main touch panel 104 serving as both of a main operation panel and a main display panel, and speaker unit 105, all of which are disposed on the front face of unit housing 106. Injection control unit 101 is wire-connected to controller unit 107 as a separate component through connector 108.

Injection head 110 is attached to the top end of caster stand 111 through movable arm 112. As shown in Fig. 2, head body 113 serving as an injector body has concave portions 114 formed as semi-cylindrical grooves in the upper surface for removably mounting liquid syringe 200.

Each concave portion 114 has cylinder-holding mechanism 116 formed in the forward section for removably holding cylinder flange 211 of liquid syringe 200 and also has piston driving mechanism 130 arranged in the rearward section for holding and sliding piston flange 221. Cylinder holding mechanism 116 is formed in concave portions 114 as a deformed reentrant groove, with which each of cylinder flanges 211 removably engages.

Since two concave portions 114 of injection head 110 receive liquid syringe 200C filled with a contrast media as a liquid and/or liquid syringe 200W filled with physiological saline as a liquid, these two concave portions 114 and two piston driving mechanisms 130 constitute a contrast media injection mechanism 130C for injecting the contrast media and a physiological saline injection mechanism 130W for injecting physiological saline into a patient.

As shown in Fig. 4, piston driver mechanism 130 has slide rod 131 as a slider member elongated in the to-and-fro direction. Slide rod 131 is supported on head body 113 slidable in the to-and-fro direction but not rotatable around the axis.

Piston press member 132 is formed integrally with slide rod 131 at the front end thereof. A pair of engaging nails 133 openable or closable as required are mounted on the left and right of piston press member 132. Slide rod 131 has screw hole 134 from the center in the rear face toward the front. A mail screw 136 to be fitted into screw hole 134 is provided for screw shaft 137 serving as a rotation member.

Screw shaft 137 is rotatably supported in the diameter direction by radial bearing 138 on head body 113, and rotatably supported in the direction of the axis by thrust bearing 139. More particularly, annular flange 141 is formed near the rear end of screw shaft 137, and annular thrust bearing 139 abuts on the rear face of flange 141.

Load cell 142 having the same shape as thrust bearing 139 and serving as a pressure detection element abuts on the rear face of thrust bearing 139. Load cell 142 is fixed to head body 113, while thrust bearing 139 is supported on head body 113 such that it can be moved slightly in the to-and-fro direction.

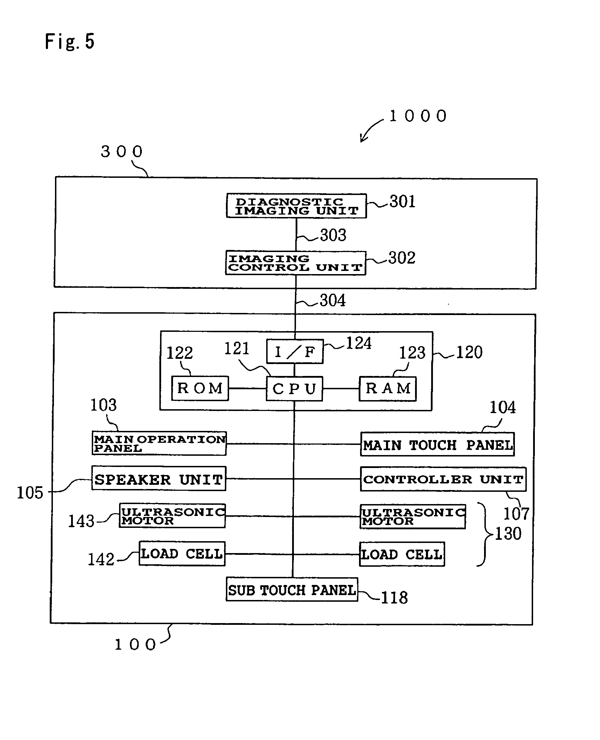

As shown in Fig. 5, piston driving mechanism 130 has ultrasonic motor 143 as a drive motor. Ultrasonic motor 143 is connected to screw shaft 137 through belt mechanism 144 as shown in Fig. 4. More particularly, belt pulley 145 is mounted integrally at the rear end of screw shaft 137, and a belt pulley is also mounted integrally on a rotor member of ultrasonic motor 143 (not shown). Endless belt is suspended on these belt pulleys 145 and the like.

In chemical liquid injector 100 of the present embodiment, respective components of injection head 110 are made of nonmagnetic material, and the portions that cannot be made of nonmagnetic material are magnetically shielded. For example, ultrasonic motor 143 is free from generation of magnetic field even when it is operated and is made of nonmagnetic metal such as phosphor bronze alloy (Cu + Sn +P), titanium alloy (Ti-6Al-4V), and magnesium alloy (Mg + Al +Zn). Load cell 142, screw shaft 137 and the like are also made of nonmagnetic metal, while belt mechanism 144, head body 113 and the like are made of nonmagnetic resin.

In chemical liquid injector 100 of the present embodiment, respective components such as main operation panel 103 and load cell 142 are connected to computer unit 120 which integrates and controls the components based on a computer program installed therein. Computer unit 120 controls the operation of piston driving mechanism 130 of injection head 110 in response to manual operation on main operation panel 103, main touch panel 104 of injection control unit 101, and/or controller unit 107, and computer unit 120 displays various data in association with the operation on main touch panel 104.

Sub touch panel 118 serving as a sub operation panel and a sub display panel is mounted on the side of injection head 110. Computer unit 120 also controls the operation of piston driving mechanism 130 of injection head 110 in response to manual operation on sub operation panel 118 and displays various data in association with the operation on sub touch panel 118.

Although described later in detail, sub touch panel 118 has a smaller screen size and fewer pixels than main touch panel 104, and thus can display a smaller amount of data. For this reason, all of the various data to be displayed is presented on main touch panel 104 but only some of them is displayed on sub touch panel 118.

As shown in Fig. 4, in chemical liquid injector 100 of the present embodiment, computer unit 120 includes hardware such as CPU (Central Processing Unit) 121, ROM (Read Only Memory) 122, RAM (Random Access Memory) 123, I/F (Interface) 124, and the like.

Computer unit 120 has a dedicated computer program installed on an information storage medium such as ROM 122 as firmware or the like, and CPU 121 executes various processes in accordance with the computer program.

Computer unit 120 recognizes data input to main/sub touch panels 104 and 108 and controls the data display. Thus, in chemical liquid injector 100 of the present embodiment, various data for normal injection is input and then output as display on main touch panel 104, while various data for test injection is input and then output as display on sub touch panel 118.

More particularly, control data for piston driving mechanism 130 dedicated to checking of the connection of extension tube 230 is registered in computer unit 120 as one of the various data for test injection. When an instruction to start test injection is input to sub touch panel 118, computer unit 120 controls the operation of ultrasonic motor 143 of physiological saline injection mechanism 130W in accordance with the control data for test injection.

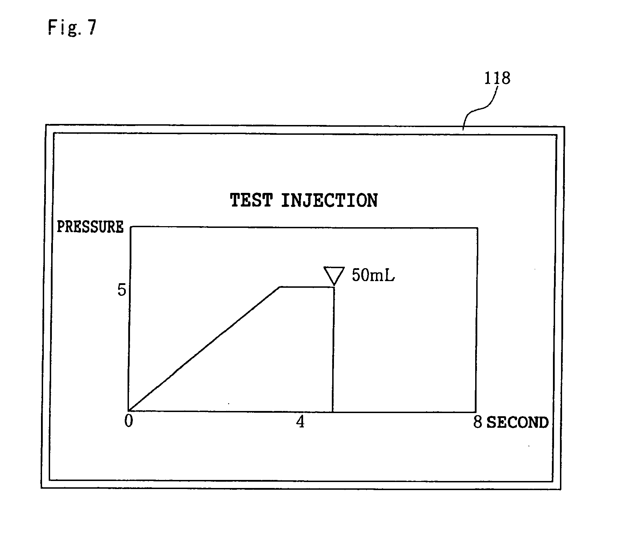

In this event, the pressure of the physiological saline injected into the patient is detected by load cell 142. As shown in Fig. 7, computer unit 120 displays a test screen with its vertical axis representing the injection pressure and its horizontal axis representing the injection time on sub touch panel 118, and outputs a graph of the detected pressure over time on the test screen in real time as one of the various data for test injection.

In addition, computer unit 120 detects the occurrence of an error if the pressure detected by load cell 142 goes out of a predetermined permissible range during the test injection. Computer unit 120 outputs the error occurrence as display on sub touch panel 118 and main touch panel 104, outputs the error occurrence as sound in speaker unit 105, and forcedly stops physiological saline injection mechanism 130W.

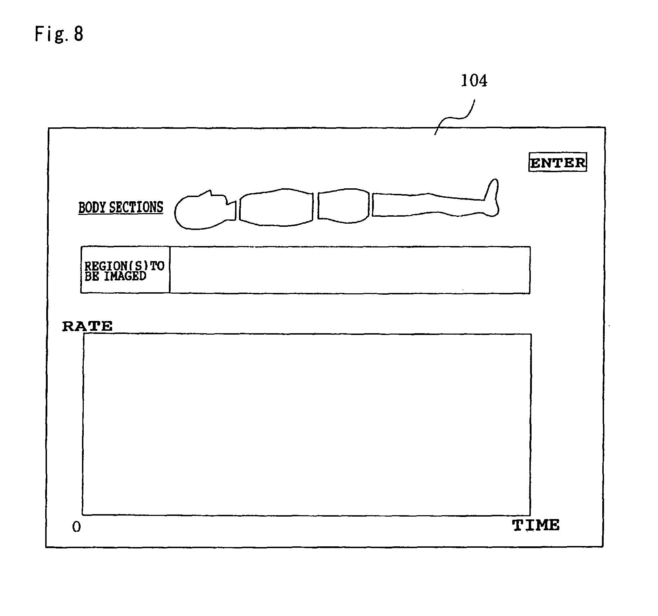

When computer unit 120 controls the operations of physiological saline/contrast media injection mechanisms 130W and 130C to perform normal injection, it displays a condition screen with its vertical axis representing the injection rate of the liquid and its horizontal axis representing the injection time on main touch panel 104 as one of the various data for normal injection as shown in Fig. 8.

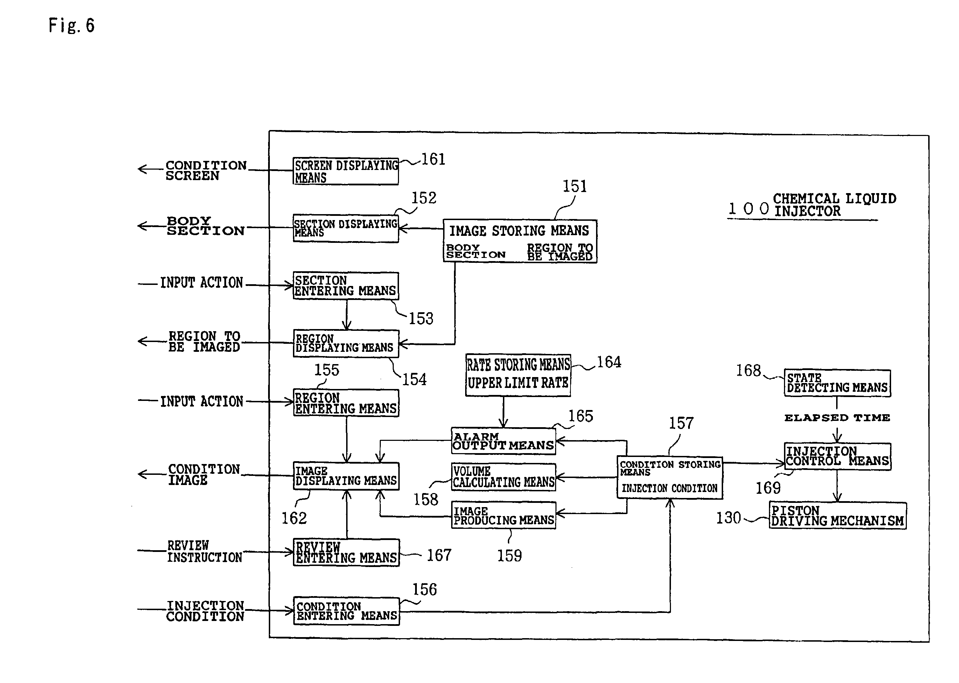

More particularly, computer unit 120 operates in accordance with the computer program installed therein as described above to allow chemical liquid injector 100 of the present embodiment to logically have, as various functions, various means including image storing means 151, section displaying means 152, section entering means 153, region displaying means 154, region entering means 155, condition entering means 156, condition storing means 157, volume calculating means 158, image producing means 159, screen displaying means 161, image displaying means 162, rate storing means 164, alarm output means 166, review entering means 167, state detecting means 168, and computer unit 149, as shown in Fig. 6.

Storing means 151, 157, ... correspond to storage areas set up in RAM 123 for CPU 121 to recognize data stored therein according to the computer program. Displaying means 152, 154, ... correspond to functions of CPU 121 to display stored data from RAM 123 on main touch panel 104.

Entering means 153, 155, ... correspond to functions of CPU 121 to recognize data based on an input action on main/sub operation panels 103 and 118. Other various functions 158, 159, ... correspond to functions of CPU 121 to process data.

Image storing means 151 stores data of schematic images of a plurality of body sections of a human body and data of schematic images of a number of regions to be imaged in relation to each other. Section displaying means 152 displays schematic images of body sections whose data are stored by image storing means 151 in the shape of a human body.

Section entering means 153 accepts an input action to select one of the body sections displayed by section displaying means 152. Region displaying means 154 displays a schematic image of at least one region to be imaged which corresponds to the body section selected by section entering means 153. Region entering means 155 accepts an input action to select the region to be imaged whose image has been displayed by region displaying means 154.

More specifically, chemical liquid injector 100 of the present embodiment defines "head part, chest part, abdomen part, and leg part" as a plurality of body sections, and schematic images corresponding to those body sections are registered as data in ROM 122. When a certain action is performed on chemical liquid injector 100 of the present embodiment, schematic images of "head part, chest part, abdomen part, and leg part" in association with body shapes are displayed on an upper screen area of main touch panel 104, as shown in Fig. 8.

Schematic images of "brain part, jaw part, and neck part" are registered as a plurality of regions to be imaged in relation to the schematic image of the body section "head part." Similarly, schematic images of "heart part and lung part" are registered in relation to the schematic image of the body section "chest part," schematic images of "stomach part, liver part, ..." are registered in relation to the schematic image of the body section "abdomen part," and schematic images of "upper part and lower part" are registered in relation to the schematic image of the body section "leg part."

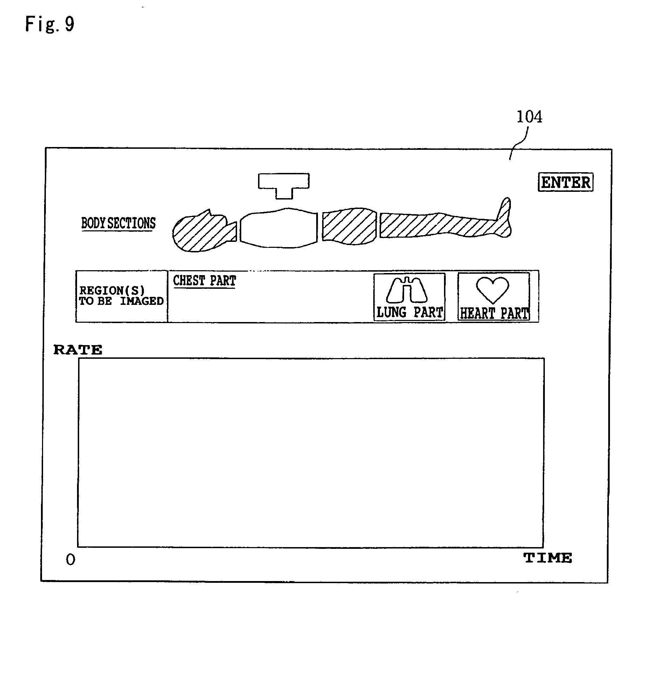

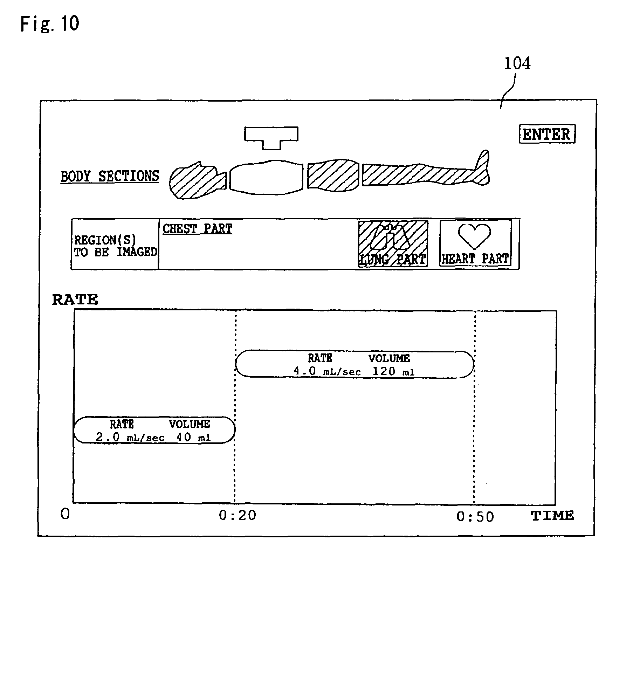

When one of the schematic images of the body sections displayed as a human body shape on main touch panel 104 is manually acted upon, a schematic image of a scanner mechanism is displayed above only the schematic image which is acted upon, and that schematic image is highlighted with the other schematic images darkened, as shown in Fig. 9. The schematic images of the regions that are related to the highlighted image are displayed below the displayed schematic images of the body sections. When one of the displayed schematic images of the related regions is manually acted upon, that schematic image is highlighted with the other schematic images darkened, as shown in Fig. 10.

Condition entering means 156 accepts an input action of an injection condition including an injection rate for each of injection time periods of a contrast media and physiological saline for the region to be imaged selected by region entering means 155. Condition storing means 157 stores the injection condition entered by condition entering means 156 for each region to be imaged.

Volume calculating means 158 calculates the injection volume from the injection time period and the injection rate for each injection condition stored by condition storing means 157. Image producing means 159 produces a condition image having a horizontal width corresponding to the injection time period and including text data which represents the injection rate and the injection quality for each stored injection condition.

As shown in Fig. 8, screen displaying means 161 displays a horizontally rectangular condition screen with its vertical axis representing the injection rate of the liquid and its horizontal axis representing the injection time period. As shown in Fig. 10, image displaying means 162 displays the condition image produced by image producing means 159 in the condition screen at the vertical position in association with the injection rate and the horizontal position in association with the injection time period.

In chemical liquid injector 100 of the embodiment, the injection conditions for the contrast media and the physiological saline are individually input as described above, so that the condition images thereof are produced in different colors such as green and blue, and the condition images are arranged horizontally and displayed in the condition screen.

When the plurality of condition images are arranged horizontally and displayed, a line segment is displayed at the position of the border between them, and a numerical value representing the injection time is displayed as text data at the lower end of the line segment. For example, when a first injection time period is "20(sec)" and a second injection time period is "30(sec)," a first injection time and a second injection time are displayed as "20(sec)" and "50(sec)," respectively.

Condition storing means 157 stores a default injection condition in advance for each region to be imaged in preparation for the case where no injection condition is input by condition entering means 156. It also stores the previous injection condition input by condition entering means 156 for each region to be imaged.

Thus, in chemical liquid injector 100 of the embodiment, when a region to be imaged is selected as described above, a condition image with the previous injection condition, if stored, is produced and displayed even when no injection condition is input for the current injection. If the previous injection condition is not stored, a condition image with the default injection condition is displayed.

The input action of the injection condition by condition entering means 156 is performed, for example, by entering numerical values on operation panel 103, and also performed by directly acting upon a condition image displayed on main touch panel 104. In this case, the operator can touch the center of the displayed condition image with a fingertip to move the condition image upward and/or downward with the fingertip to increase and/or reduce the injection rate of the injection condition.

In addition, the operator can touch at least one of both lateral ends of the displayed condition image with a fingertip and moves the fingertip leftward and/or rightward to increase and/or reduce the injection time period and/or the injection volume of the injection condition. When the operator touches with a fingertip the numerical value of the injection rate or the injection volume of the displayed condition image, an image of a numeric keypad is displayed there (not shown), and the operator can increase and/or reduce the injection rate and/or the injection volume by manually operating the numeric keypad. The data of the stored injection condition is updated in response to the manual operation of the condition image as described above, and the data of the displayed condition image is updated in real time.

Rate storing means 164 stores the upper limit rate of liquid injection in advance. Alarm output means 166 outputs an alarm when the injection rate of the injection condition stored by condition storing means 167 exceeds the upper limit rate. More specifically, the upper limit rate is registered as data for each region to be imaged and each type of liquid, and is read out in association with the selected region to be imaged and type of liquid. The condition image which includes the injection rate exceeding the upper limit rate blinks in a dedicated color such as red, and a predetermined guidance message as "critical rate" is provided as alarm display.

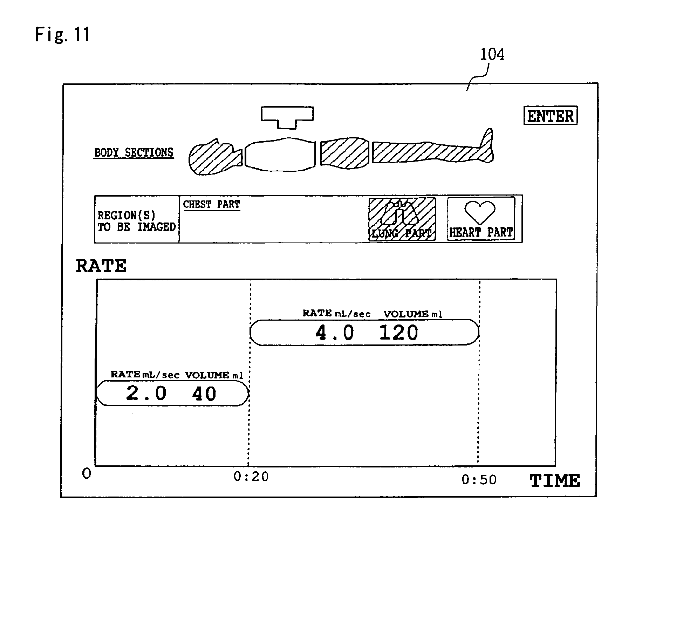

Review entering means 167 accepts an input action of a review instruction. Image displaying means 162 enlarges the text data of the condition image when the review instruction is entered as shown in Fig. 11. The review instruction may be input on main touch panel 104 on which the injection condition is displayed, but it is normally entered on sub operation panel 118 of injection head 110 separate from main touch panel 104.

As shown in Fig. 10, the condition image is displayed as a horizontal rectangle having semicircular shape at both ends in the embodiment. Generally, item names as "rate" "volume" are displayed as text data at predetermined positions in upper portions inside the rectangle, and the numerical values and their units as "2.0mL/sec" "4.0ml" are displayed as text data under the associated item names.

When the review instruction is entered as described, as shown in Fig. 11, the text data representing the item name as "rate" and the unit as "mL/sec" is displayed over the condition image, and only the text data representing the numerical value as "2.0" is enlarged in the condition image.

When liquid injection is performed in response to manual operation on main touch panel 104 or the like, state detecting means 168 measures the elapsed time from the start of the injection. Computer unit 149 sequentially controls the operations of the plurality of physiological saline/contrast media injection mechanisms 130W and 130C in real time based on the elapsed time measured by state detecting means 168 and the plurality of stored injection conditions.

While the above various means of chemical liquid injector 100 are accomplished by pieces of hardware such as main touch panel 104 as required, they are mainly implemented by CPU 121 as a piece of hardware functioning in accordance with resources and computer programs stored on an information storage medium such as ROM 122.

Such resources are formed of, for example, a data file including schematic images of a plurality of body sections of a human body and schematic images of a number of regions to be imaged in relation to each other, a data file including injection conditions of contrast media/physiological saline injection mechanisms 130C and 130W for each of the number of regions to be imaged of a human body, a data file including the upper limit rates of contrast media/physiological saline injection mechanisms 130C and 130W for each region to be imaged, and the like.

The abovementioned computer program is stored on an information storage medium such as RAM 123 as software for causing CPU 121 or the like to perform processing operations including as display of schematic images of the plurality of body sections registered as data in RAM 123 or the like in the shape of a human body on main touch panel 104 and display of a horizontally rectangular condition screen, under the schematic images, with the vertical axis representing the injection rate of the liquid and the horizontal axis representing the injection time period, reception of an input action on main touch panel 104 to select one of the plurality of body sections displayed as the image, display of the schematic image of at least one region to be imaged in association with the selected body section, reception of an input action to select the region to be imaged displayed as the image, display of the injection condition registered as data in association with the selected region to be imaged together with the condition screen, reading of the previous injection conditions of the contrast media and physiological saline for the selected region to be imaged from RAM 123 or the like, reading of the default injection condition when the previous injection condition is not stored, calculation of the injection volume from the injection time period and the injection rate for each of the stored injection conditions, production of the condition image having the horizontal width corresponding to the injection time period and including the injection rate and the injection volume as text data for each of the stored injection conditions, display of the produced condition image in the condition screen at the vertical position in association with the injection rate and the horizontal position in association with the injection time period, reception of an input action of the injection condition as edit operation of the displayed condition image, storage of the newly input injection condition to reflect it on the data production for the condition image and display thereof, output of an alarm when the injection rate of the stored injection condition exceeds the upper limit rate, enlargement of the text data of the condition image displayed on main touch panel 104 when a review instruction is entered on the sub operation panel 118 or the like, performance of the liquid injection in response to manual operation on main touch panel 104 or the like, measurement of the elapsed time from the start of the injection, and sequential control of the operations of the plurality of physiological saline/contrast media injection mechanisms 130W and 130C in real time based on the elapsed time and the plurality of stored injection conditions.

### <Operation of the Embodiment>

For using chemical liquid injector 100 of the embodiment in the above construction, injection head 110 of chemical liquid injector 100 is disposed near imaging unit 301 of MRI apparatus 300, and injection control unit 101 is placed at a predetermined position away from imaging unit 301 as shown in Fig. 3

In this state, for example, the operator (not shown) moves to near injection head 110 and connects liquid syringes 200C and 200W for the contrast media and physiological saline to the patient (not shown) at the position of imaging unit 301 through bifurcated extension tube 230 as shown in Fig. 2. Cylinder members 210 of liquid syringes 200 are held in concave portions 114 of injection head 110, and piston members 220 are held by piston driving mechanisms 130.

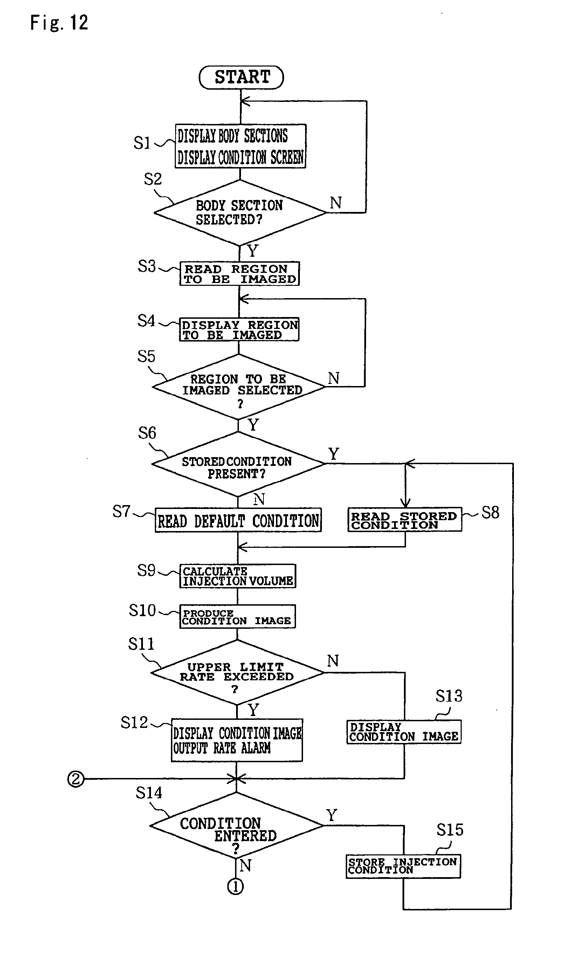

Next, the operator moves to near injection control unit 101 from near injection head 110 to activate chemical liquid injector 100 by an input action on main operation panel 103 or the like. Then, as shown in Figs. 8 and 12, schematic images of a plurality of body sections are displayed in the shape of a human body in an upper portion of main touch panel 104, and under the schematic images, a horizontally rectangular condition screen is displayed with its vertical axis representing the injection rate of the liquid and its horizontal axis representing the injection time period (step S1).

If the operator presses, with a fingertip, one of the schematic images of the body sections displayed on main touch panel 104 (step S2), then, as shown in Fig. 9, only the selected schematic image of the body section is highlighted with the other schematic images darkened, and a schematic image of a scanner mechanism is displayed above the selected schematic image of the body section.

At the same time, schematic images of a plurality of regions to be imaged which are related to the selected body section are read and displayed below the displayed schematic images of the body sections (steps S3, S4). If the operator presses one of the schematic images of the regions (step S5), only the selected schematic image of the region is highlighted with the other schematic images darkened as shown in Fig. 10.

When the region to be imaged is selected as described above, chemical liquid injector 100 of the embodiment checks whether the previous injection condition associated with the selected region to be imaged is registered as data in RAM 123 (step S6). If the data is not registered, the default injection condition is read out (step S7). If the data is registered, the previous injection condition is read out (step S8). In chemical liquid injector 100 of the embodiment, the contrast media and the physiological saline are basically injected sequentially, so that the injection conditions of the contrast media and the physiological saline are registered in association with the region to be imaged, and these injection conditions are read out.

Since the injection conditions thus read out include the injection time period and the injection rate set as data, the injection time period is multiplied by the injection rate to calculate the injection volume for each injection condition (step S9). Then, a condition image is produced for each injection condition to have a horizontal width corresponding to the injection time period and to include the injection rate and injection volume as text data (step S10). As shown in Fig. 10, the condition image is displayed in the condition screen at the vertical position in association with the injection rate and the horizontal position in association with the injection time period (steps S12, S13).

Since the condition images are produced from the injection conditions of the contrast media and physiological saline as described above, the condition images are displayed in green and blue, respectively, and arranged horizontally. It is determined whether or not the injection rate exceeds the upper limit rate for each injection condition (step S11). For the injection condition with the excessive injection rate, the condition image blinks in red to output a rate alarm (step S12).

In chemical liquid injector 100 of the embodiment, the injection condition can be changed as desired even in that state (steps S13, S14). In that case, for example, the operator can touch the center of the condition image displayed on main touch panel 104 with a fingertip to move the condition image upward and/or downward with the fingertip to increase and/or reduce the injection rate of the injection condition.

In addition, the operator can touch at least one of both of lateral ends of the displayed condition image with a fingertip and moves the fingertip leftward and/or rightward to increase and/or reduce the injection time period and/or the injection volume of the injection condition. When the operator touches with a fingertip the numerical value of the injection rate and/or the injection volume of the displayed condition image, an image of a numeric keypad is displayed there (not shown), and the operator can increase and/or reduce the injection rate and/or the injection volume by manually operating the numeric keypad.

When the condition image is manually acted upon to enter the injection condition in this manner (step S14), that is stored as the current injection condition (step S15), and the data production of the condition image is performed in real time (steps S8 to S14).

In ordinary operation with chemical liquid injector 100 of the embodiment, the operator moves from near injection control unit 101 to near injection head 110 after the data setting for normal injection is completed as described above, and then performs the test injection.

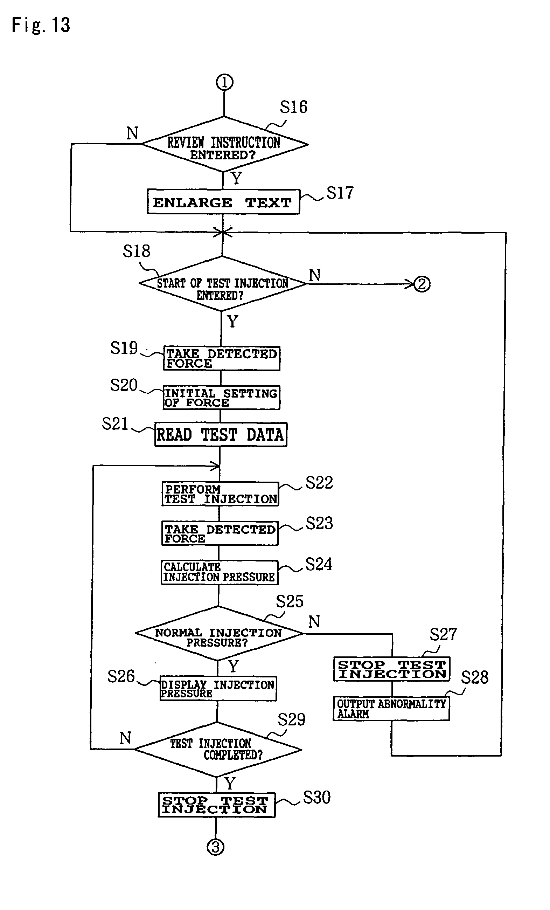

Since the operator stays away from injection control unit 101, he or she does not easily check visually the injection condition in the condition image displayed on main touch panel 104. Thus, when the operator wishes to review the injection condition, the operator enters a review instruction on sub operation panel 118 of injection head 110 as shown in Fig. 13 (step S16).

Then, as shown in Fig. 11, the text data of the numerical values of the injection rate and injection volume displayed in the condition image is enlarged (step S17). The operator can easily review the injection condition of the condition image displayed on main touch panel 104 of injection control unit 101 away from the operator.

When the operator near injection head 110 makes an input action to start the test injection on sub touch panel 118 (step S18), computer unit 120 first takes an electric resistance as an electric signal from load cell 142 without driving physiological saline/contrast media injection mechanisms 130W and 130C (step S19), and performs initial setting, assuming that the electric resistance shows the force of "zero" (step S20).

Next, computer unit 120 reads out the control data for test injection (step S21), and performs test injection by activating ultrasonic motor 143 only in physiological saline injection mechanism 130W in accordance with the control data (step S22).

Screw shaft 137 connected to ultrasonic motor 143 through belt mechanism 144 as shown in Fig. 4 is driven to rotate. Since screw shaft 137 is supported in the axis direction through thrust bearing 139 by load cell 142 fixed to head body 113, slide rod 131 connected to screw shaft 137 through the screw mechanism is slid.

Slide rod 131 presses piston member 220 into cylinder member 210, and the liquid is injected from cylinder member 210 into the patient. At this point, the force acting on slide rod 131 pressing piston member 220 also acts on screw shaft 137, so that the press acting on screw shaft 137 also acts on load cell 142 through thrust bearing 139.

As shown in Fig. 13, computer unit 120 takes the force detected by load cell 142 during the liquid injection as described above (step S23), and calculates the injection pressure from the detected force in association with the initially set viscosity of the liquid, the internal diameter of cylinder member 210 and the like (step S24).

It is checked whether or not the calculated injection pressure is normal or not (step S25). As shown in Fig. 7, the injection pressure is displayed on sub touch panel 118 if it is normal (step S26). The operator can check the pressure of the physiological saline in the test injection in real time while he or she stays near injection head 110.

If the calculated injection pressure is not normal in chemical liquid injector 100 (step S25), the operation of ultrasonic motor 143 is forcedly stopped (step S27), and the occurrence of abnormality is notified with display output on main/sub touch panels 104 and 118 and sound output from speaker unit 105 (step S28).

More specifically, computer unit 120 stores data of the injection pressure when the liquid is normally injected into a patient from cylinder member 210,data of the injection pressure when air is mixed in the liquid, and data of the injection pressure when air is injected into a patient from cylinder member 210. Computer unit 120 forcedly stops physiological saline injection mechanism 130W if it does not detect the data which shows the normal injection of the liquid, the data which shows the mixing of air into the liquid, and the data which shows the injection of air.

The occurrence of abnormality is notified with the display output on main/sub touch panels 104 and 118 and the sound output from speaker unit 105 as an abnormality alarm such as "Abnormal pressure is detected. Check syringe and tube," "Air mixing is detected. Check syringe and tube," "Air injection is detected. Check patient," and the like. The operator quickly and appropriately recognizes and deals with the occurrence of abnormality.

The data of optimal injection pressure is set in the initial setting of the operation mode. When the test injection is performed as described above (steps S22 to S29), the output from ultrasonic motor 143 is controlled in feedback to cause the calculated injection pressure to be equal to the set optimal pressure (step S22).

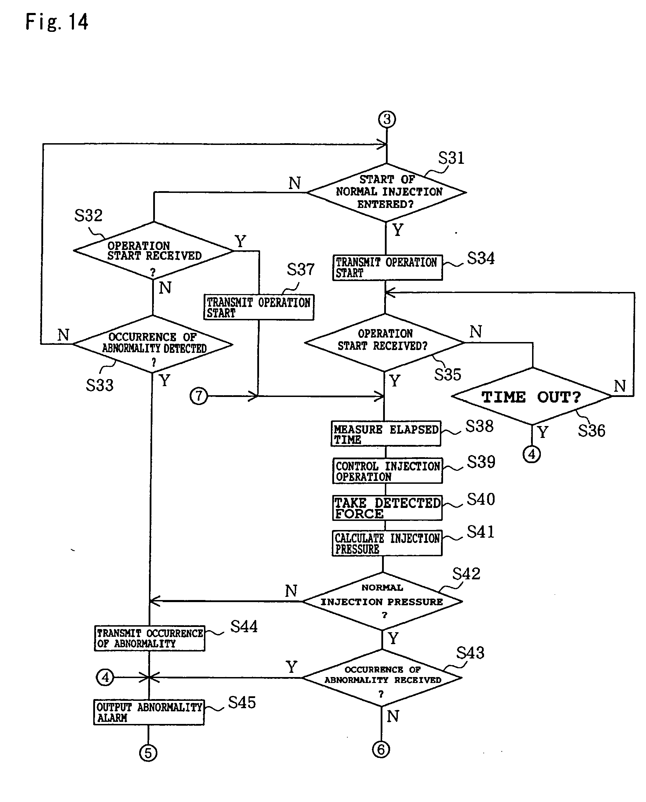

The abovementioned operation is repeated (steps S22 to S29) until the injection completion is detected from the comparison between the operation distance of physiological saline injection mechanism 130W and the effective distance of piston member 220 or the like (S29). If the test injection is completed without problems (step S29), the operation of ultrasonic motor 143 is stopped (step S30), and chemical liquid injector 100 of the present embodiment is ready for the normal injection as shown in Fig. 14.

The operator moves from near injection head 110 to near injection control unit 101 and makes an input action to start the normal injection on main touch panel 104. Upon detection of that (step S31), chemical liquid injector 100 transmits data indicating the start of operation to MRI apparatus 300 (steps S31 and S34).

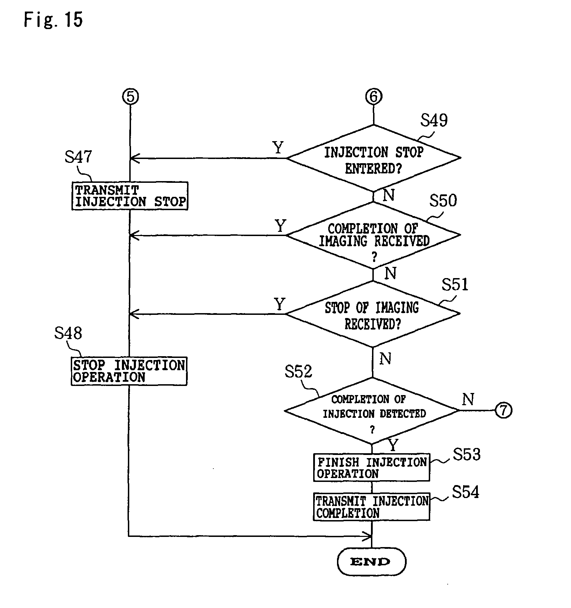

As shown in Fig. 15, MRI apparatus 300 receives the data indicating the start of operation from chemical liquid injector 100 (step T2), transmits the data indicating the start of the operation back to chemical liquid injector 100, and performs imaging operation (step T8). Thus, in imaging diagnostic system 1000 of the present embodiment, the imaging of MRI apparatus 300 follows the liquid injection of chemical liquid injector 100.

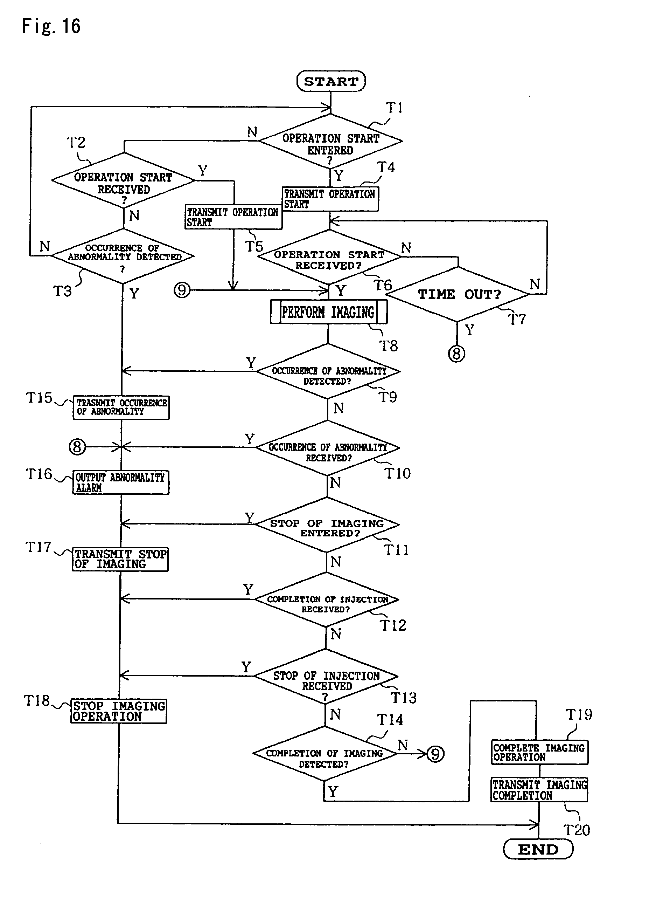

In imaging diagnostic apparatus 1000 of the present embodiment, if the operator makes an input action to start imaging on MRI apparatus 300 (step T1) as shown in Fig. 16 when chemical liquid injector 100 is ready as described above (steps S31 to S33), the liquid injection of chemical liquid injector 100 follows the imaging of MRI apparatus 300 (steps T4, T6 and afterward, S32, S37 and afterward).

When chemical liquid injector 100 performs a series of operations for liquid injection as shown in Fig. 14 (step S38 and afterward), the elapsed time from the start of injection is measured (step S38). The operations of contrast media injection mechanism 130C and physiological saline injection mechanism 130W are sequentially controlled in real time in accordance with the elapsed time and the read injection conditions (step S39).

Again, computer unit 120 takes the force detected by load cell 142 (step S40), calculates the injection press from the detected force in association with the initially set viscosity of the liquid or internal diameter of cylinder member 210 (step S41), and checks if the injection pressure is normal or not (step S42).

In chemical liquid injector 100 or MRI apparatus 300 of the present embodiment, if the occurrence of abnormality is detected in the ready state described above (Steps S33, T3), or the occurrence of abnormality is detected during the operation (steps S42, T9), then the occurrence of abnormality is notified (steps S45, T16) and the operation is stopped (steps S48, T18).

Since the occurrence of abnormality is notified also to the other apparatus (steps S44, T15), the other apparatus which has received that notification (steps T10, S43) also provides a notification of the occurrence of abnormality (steps T16, S45). Since the stop of operation in one apparatus is also notified to the other apparatus (steps *S47, T17), the other apparatus which has received that notification (steps T13, S51) also stops its operation (steps T18, S48).

When an input action to stop the operation is made in one apparatus (steps S49, T11), the operation is stopped in the apparatus (steps S48, T18) and that is notified to the other apparatus (steps S47, T17). As a result, the other apparatus which has received that notification (steps T13, S51) stops its operation (steps T18, S48).

Furthermore, when the completion of the injection is detected in one apparatus (steps S52, T14), the operation is completed in the apparatus (steps S53, T19) and that is notified to other apparatus (steps S54, T20). As a result, the other apparatus which has received the notification (steps T12, S51) stops its operation (steps T18, S48).

### <Effect of the Embodiment>

In chemical liquid injector 100 of the present embodiment, the various data for normal injection is input and then output as display on large and high-resolution main touch panel 104 mounted on injection control unit 101 as described above. Thus, the complicated input operation and data display for the normal injection can be performed appropriately.

In addition, since the various data for test injection is input and then output as display on small and low-resolution sub touch panel 118 mounted on injection head 110, a single operator can easily input the data for test injection and check the data while he or she visually checks extension tube 230 and the like near injection head 110.

In chemical liquid injector 100 of the present embodiment, the pressure of the physiological saline injected into the patient in the test injection is detected by load cell 142 and displayed in real time on sub touch panel 118. The operator can easily and reliably check the pressure of the physiological saline injected into the patient in the test injection.

If the pressure detected during the test injection goes out of the predetermined permissible range, computer unit 120 detects the occurrence of an error which is then displayed on sub touch panel 118, so that the operator can quickly see and deal with the abnormal pressure in the test injection.

In chemical liquid injector 100 of the present embodiment, piston driving mechanism 130 is supported movably on head body 113, and load cell 142 for detecting the force acting on piston driving mechanism 130 is fixed to head body 113. Since load cell 142 is not disposed in the movable portion of piston driving mechanism 130, the structure is simple and provides excellent manufacturability and reliability

Particularly, in piston driving mechanism 130, slide rod 131 pressing piston member 220 is supported slidably and non-rotatably, and load cell 142 detects the force acting in the axis direction on rotatable and non-slidable screw shaft 137 connected to slide rod 131 through the screw mechanism. Load cell 142 can reliably detect the force acting on piston press member 220 with a simple structure.

Since screw shaft 137 is supported in the diameter direction by radial bearing 138 and supported in the axis direction by thrust bearing 139, it can be rotated smoothly with a simple structure. Load cell 142 detects the force acting in the axis direction on thrust bearing 139, so that load cell 142 can detect the force acting on piston press member 220 without inhibiting the rotation of screw shaft 137.

Ultrasonic motor 143 and screw shaft 137 are connected to each other through belt mechanism 144, so that screw shaft 137 can be moved in the axis direction without preventing the power transmission, and load cell 142 can favorably detect the force acting on screw shaft 137.

As shown in Fig. 10, in chemical liquid injector 100 of the embodiment, the condition image for the injection condition including the injection rate as text data and having the horizontal width corresponding to the injection time period is displayed in the condition screen with the vertical axis representing the injection rate and the horizontal axis representing the injection time period at the vertical position in association with the injection rate and the horizontal position in association with the injection time period on main touch panel 104.

With the displayed image on large and high-resolution main touch panel 104, the operator easily understands instinctively the injection condition from the horizontal width and the position of the condition image. Also, since the text data of the injection rate is included in the condition image, the operator can quickly review the numeric values.

In addition, the operations of piston driving mechanisms 130 are controlled in real time based on the injection condition and the elapsed time, so that the liquid can be injected into the patient at the appropriate rate for the appropriate time period. Particularly, since the injection conditions of the contrast media and physiological saline are automatically arranged sequentially, contrast media/physiological saline injection mechanisms 130C and 130W can automatically be operated sequentially to inject the contrast media and physiological saline into the patient in an appropriate order.

Since only the injection time period and the injection rate are necessary for the input action of the injection condition, the input action is simple and easy. Particularly, the injection condition can be registered as data for each region to be imaged of a human body, and when a region to be imaged is selected by an input action, the previous injection condition is automatically read out to display the condition image including that injection condition. The injection condition does not need to be entered each time and the liquid can be injected under an appropriate condition for each region to be imaged.

When the previous injection condition is not registered as data, the default injection condition is read out to display the condition image including the default condition. The liquid can be injected under an appropriate injection condition for each region to be imaged even when the injection condition is not input. The input action of the injection condition can be performed by manually acting upon the displayed previous condition image or default condition image, so that the input action is extremely simple and easy.

Particularly, since the condition image is displayed on main touch panel 104 and manually acted upon, the operator can directly act upon the condition image manually Also, since the operator can touch the center of the displayed condition image with a fingertip to move the condition image upward and/or downward with the fingertip to increase and/or reduce the injection rate of the injection condition, the injection rate can be changed simply and easily.

The operator can touch at least one of both of lateral ends of the displayed condition image with a fingertip and moves the fingertip leftward and/or rightward to increase and/or reduce the injection time period and the injection volume of the injection condition. Thus, the injection time period and the injection volume can be changed simply and easily. When the operator touches with a fingertip the numerical values of the injection rate and the injection volume of the displayed condition image, an image of a numeric keypad is displayed there, and the operator can manually operate the numeric keypad to simply and easily change the injection rate and the injection volume.

In addition, when the injection rate and the injection time period are input as described above, the injection volume is automatically calculated and provided as text data in the condition image, so that the operator can see the injection volume only by inputting the injection rate and the injection time period. When the operator enters a review instruction to chemical liquid injector 100, the text data of the condition image is enlarged, allowing the operator to favorably review the injection rate and the injection volume.

Particularly, in chemical liquid injector 100 of the embodiment, injection control unit 101 on which the condition image is displayed is separate from injection head 110 on which the check operation is performed, but when a review instruction is entered to injection head 110, the text data of the condition image displayed on injection control unit 101 is enlarged. The operator performing the various operations in the test injection on injection head 110 can favorably review the injection rate and the injection volume of the injection condition displayed on injection control unit 101.

As shown in Fig. 10, the text data of the condition image is produced as a combination of the numeric value with its unit. However, as shown in Fig. 11, only the text data of numerical value is enlarged, and the text data of the unit is displayed outside the condition image. Thus, only the required information can be enlarged without unnecessarily enlarging the whole condition image.

Since chemical liquid injector 100 of the embodiment displays the condition images of the contrast media and the physiological saline in the individually dedicated colors, the operator can make a clear distinction between the injection conditions of the contrast media and the physiological saline. In addition, since the rate alarm is output when the injection rate of the injection condition exceeds the upper limit rate, it is possible to prevent the liquid from being injected into the patient at a critical rate.

Since the rate alarm is performed by displaying the condition image in the alarm color and blinking it, it is possible to simply and reliably notify the operator that the injection rate is at a critical rate. In addition, the injection conditions of the contrast media and the physiological saline are normally displayed in blue and green, but they are displayed in the complementary color or red when the rate thereof is at a critical rate, so that the operator can be notified clearly that the injection rate is at a critical rate.

In chemical liquid injector 100 of the embodiment, the schematic images of a plurality of body sections are displayed in the shape of a human body on main touch panel 104. When the operator manually acts upon one of them as desired, the schematic images of the plurality of regions to be imaged associated with the body section acted upon are displayed. When the operator manually acts upon one of them as desired, the single region to be imaged is selected. Thus, the selection of a region to be imaged for use in data registration or data reading for the injection condition can be performed reliably with simple operation.

Since schematic images of a plurality of body sections are displayed in the shape of a human body, the operator can select any of the body sections easily and reliably. Since schematic images of body sections and regions to be imaged are displayed on main touch panel 104 and can directly be manually acted upon, they can be selected easily and reliably.

In imaging diagnostic system 1000 of the present embodiment, since the liquid injection performed by chemical liquid injector 100 and the shooting of images made by MRI apparatus 300 are automatically linked to each other, diagnostic images can be taken in appropriate timings from the patient injected with the contrast media and physiological saline sequentially in proper timings.

### <Modifications of Embodiment>

The present invention is not in any way limited to the above-described embodiment, but various changes or modifications may be made without departing from the scope of the invention. For example, although chemical liquid injector 100 according to the above embodiment has contrast medium/physiological saline injection mechanisms 130C and 130W for injecting the contrast media and physiological saline, the present invention is also applicable to a chemical liquid injector having one piston driving mechanism 130 for injecting a contrast media only (not shown).

Although the test injection is performed with the physiological saline in chemical liquid injector 100 having contrast media/physiological saline injection mechanisms 130C and 130W according to the above embodiment, the test injection can be performed with the contrast media. In that case, for example, control data for piston driving mechanism 130 dedicated to checking of a time period required for the contrast media to reach the affected area to be imaged may be registered as one of the various data for test injection in computer unit 120 to control the operation of piston driving mechanism 130 in accordance with the control data.

In addition, main/sub touch panels 104 and 118 are used to achieve the input action and data display for the normal/test injection in the above embodiment. Alternatively, for example, independent main/sub display panels and main/sub operation panels can be used to realize an input action and data display for normal/test injection, respectively (not shown).

In chemical liquid injector 100 of the above embodiment, a single injection condition is input for a single liquid. A plurality of injection conditions may be input for a single liquid. In this case, since the injection rate of the contrast media may be variable in a plurality of levels, the injection operation can be performed more precisely. In addition, the liquid injection may be automatically stopped temporarily for a predetermined time period by setting the injection rate of "zero" in the plurality of injection conditions.

In the above embodiment, the condition screen and/or the schematic images of body sections registered electronically as data are displayed on main touch panel 104. However, the condition screen and/or the schematic images of body sections may be formed fixedly, for example by painting at a predetermined position on the surface of main touch panel 104.

In the above embodiment, the data of an injection condition is registered for each region to be imaged, and then read for a desired region to be imaged in controlling the injection of a liquid. There are various other conditions than the selection of a region to be imaged for achieving optimum injection of a liquid such as a contrast media.

For example, the actual contrast media for use on MRI apparatus 300 contains an effective component of iodine whose concentration differs from product to product. Imaging conditions differ with body weights of patients to be imaged. As disclosed in Japanese patent application No. 2003-039756 filed by the present applicant, the data of the weight of a patient and the type of a contrast media used may be entered into the chemical liquid injector, and an injection condition may be adjusted depending on the entered data.

In the above embodiment, the contrast media and physiological saline are sequentially injected according to the injection condition. However, as disclosed in Japanese patent application No. 2002-363675, it is possible to dilute a contrast media with physiological saline and inject the diluted contrast media according to the injection condition.

In the above embodiment, the injection condition is entered manually to chemical liquid injector 100. The data of the injection condition may be registered on an information storage medium such a PC card in advance and the injection condition may be downloaded from the information storage medium to chemical liquid injector 100.

The data of the injection condition may be registered in an external database server, and chemical liquid injector 100 may download the registered injection condition on-line from the external database server. Similarly, the data of the injection condition may be registered in a host computer of the manufacturer of chemical liquid injector 100, and the registered injection condition may be downloaded from the host computer through the Internet to chemical liquid injector 100 which is installed in a medical facility.

In the above embodiment, the data of the default injection condition recommended by the manufacturer is registered in chemical liquid injector 100. However, it is possible to manufacture and sell chemical liquid injector 100 with no injection condition registered. In addition, although chemical liquid injector 100 stores and automatically displays the previous injection condition in the above embodiment, such data storage may not be performed, and the data of the injection conditions provided from several injections may be stored and selectively displayed by manual operation.

In the above embodiment, the horizontal axis of the condition screen represents the injection rate, and the injection condition includes the injection rate and the injection time period. The horizontal axis of the condition screen may represent the injection volume, the injection condition may include the injection rate and the injection volume, and the condition image may have a horizontal width corresponding to the injection volume and include the injection rate as text data. In this case, the chemical liquid injector may automatically calculate the injection time period from the injection rate and the injection volume and provide the injection time period as text data in the condition image.

In the above embodiment, when the injection rate in the normal injection exceeds the upper limit rate, the color of the condition image is changed and blinked to output a rate alarm. For example, a guidance message as "Critical rate, check the setting" may be provided as an alarm display in the condition image, and/or a dedicated icon may be provided as an alarm image.

As shown in Fig. 7, in the above embodiment, the pressure in the test injection is displayed in real time on sub touch panel 118 as the graph over time, and if the pressure goes out of the predetermined permissible range, the error occurrence is detected. Such error detection is performed after the detected pressure reaches the set pressure and stays the same level in the graph over time. However, it is assumed that the pressure may be steeply changed and the test injection should be stopped in the process of the detected pressure increasing toward the set pressure.

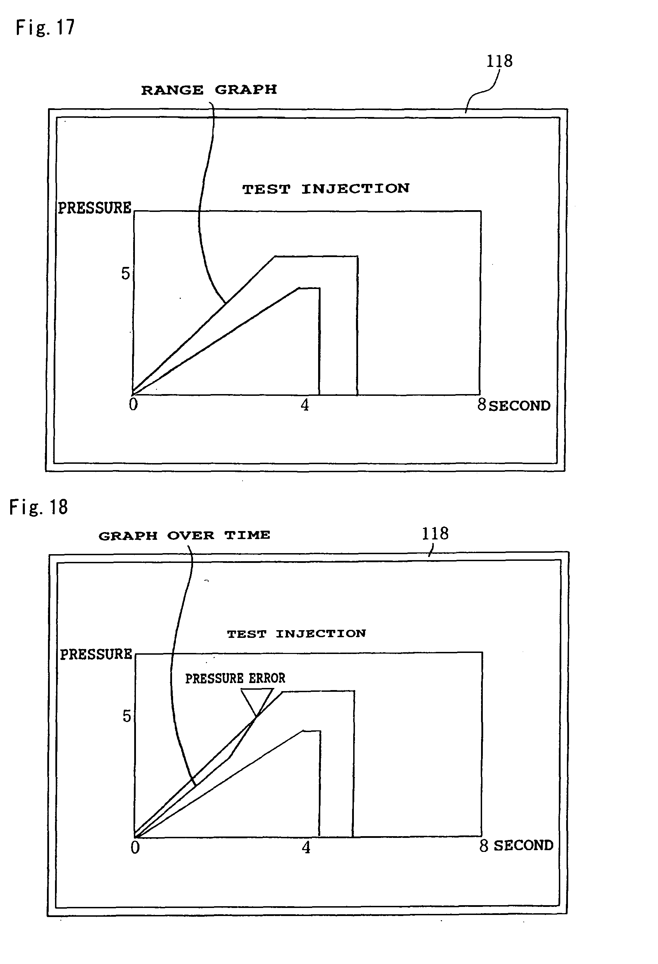

To address this, it is preferable that data of a predetermined range graph is registered in advance which represents a permissible range of pressure changing over time in the test injection as shown in Fig. 17, and occurrence of an error is detected when the graph over time produced in real time during the test injection goes out of the range graph as shown in Fig. 18.

It is possible that the abovementioned injection pressure is displayed in real time and occurrence of an error is detected when it goes out of the permissible range, or the graph of the injection pressure over time is displayed in real time and occurrence of an error is detected when it goes out of the range graph on main touch panel 104 or the like in the normal injection (not shown).

In chemical liquid injector 100 of the present embodiment, as shown in Figs. 8 to 11, the injection rate for each injection time period is displayed for the contrast media and physiological saline on main touch panel 104 in the normal injection. When the injection pressure and the graph over time are also displayed as described above, it is preferable, for example, that the screen of main touch panel 104 is divided into two to display the injection rate and the injection pressure side by side, or that the injection pressure is superimposed on the graph screen for the injection rate (not shown).

In the above embodiment, MRI apparatus 300 is used as the imaging diagnostic apparatus, and chemical liquid injector 100 injects the contrast media for use in the MRI apparatus into the patient. A CT scanner or a PET apparatus may be used as the imaging diagnostic apparatus and chemical liquid injector 100 may inject a contrast media for use in such an apparatus into a patient.

In the above embodiment, CPU 121 operates according to the computer program stored in RAM 123 or the like to logically perform the various means as the various functions of chemical liquid injector 100. The above means may be implemented by pieces of hardware, or some of the means may be stored as software in RAM 123 or the like and the others implemented by pieces of hardware.

## Claims

1. A chemical liquid injector for injecting a liquid from a liquid syringe including a cylinder member and a piston member slidably inserted into the cylinder member into a patient through an extension tube in test injection and then injecting the liquid in normal injection, comprising:
a cylinder holding mechanism for removably holding the cylinder member of the liquid syringe;
a piston driving mechanism for sliding the piston member of the held liquid syringe;
a computer unit for controlling the operation of the piston driving mechanism;
a main display panel for displaying various data as output from the computer unit;
a main operation panel for accepting an input action of the various data to the computer unit;
a sub display panel for displaying various data as output from the computer unit, the sub display panel being smaller than the main display panel;
a sub operation panel for accepting an input action of the various data to the computer unit, the sub operation panel being smaller than the main operation panel;
an injection control unit equipped with at least the computer unit, the main display panel, and the main operation panel; and
an injection head formed as a separate body from the injection control unit and equipped with at least the cylinder holding mechanism, the piston driving mechanism, the sub display panel, and the sub operation panel,
wherein various data for the normal injection is input on the main operation panel and displayed on the main display panel, and
various data for the test injection is input on the sub operation panel and displayed on the sub display panel.

2. A chemical liquid injector according to claim 1, wherein control data for the piston driving mechanism dedicated to checking of the connection of the extension tube is stored in the computer unit as the various data for the test injection,
the sub operation panel accepts an input action of an instruction to start the test injection, and
the computer unit controls the operation of the piston driving mechanism in accordance with the control data in response to the input action of the instruction to start the test injection.

3. A chemical liquid injector according to claim 2, further comprising a pressure detecting element for detecting a pressure of the liquid injected into the patient,
wherein the sub display panel displays the pressure detected during the test injection in real time as the various data for the test injection.

4. A chemical liquid injector according to claim 3, wherein the computer unit produces data of a graph over time from the pressure detected during the test injection in real time, and
the sub display panel displays the graph over time as the various data for the test injection in real time.

5. A chemical liquid injector according to claim 3 or 4, wherein the computer unit detects occurrence of an error when the pressure detected during the test injection goes out of a predetermined permissible range, and
the sub display panel displays the detected occurrence of error.

6. A chemical liquid injector according to claim 3, wherein the computer unit detects occurrence of an error when the graph over time produced in real time during the test injection goes out of a predetermined range graph corresponding to a permissible range of the pressure changing over time in the test injection, and
the sub display panel displays the detected occurrence of error.

7. A chemical liquid injector according to any one of claims 3 to 6, wherein the main display panel displays the pressure detected during the normal injection in real time as the various data for the normal injection.

8. A chemical liquid injector according to claim 7, wherein the computer unit produces data of a graph over time from the pressure detected during the normal injection in real time, and
the main display panel displays the graph over time as the various data for the normal injection in real time.

9. A chemical liquid injector according to claim 7 or 8, wherein the computer unit detects occurrence of an error when the pressure detected during the normal injection goes out of a predetermined permissible range, and
the main display panel displays the detected occurrence of error.

10. A chemical liquid injector according to claim 8, wherein the computer unit detects occurrence of an error when the graph over time produced in real time during the normal injection goes out of a predetermined range graph corresponding to a permissible range of the pressure changing over time in the normal injection, and
the main display panel displays the detected occurrence of error.

11. A chemical liquid injector according to any one of claims 3 to 10, wherein the pressure detecting element detects a force acting on the piston driving mechanism fixed to the injection head and pressing the piston member.

12. A chemical liquid injector according to any one of claims 1 to 11, wherein the liquid syringe includes a contrast media syringe for injecting a contrast media as the liquid into the patient whose image is taken by an imaging diagnostic apparatus and a physiological saline syringe for injecting physiological saline as the liquid into the patient,
the contrast media syringe and the physiological saline syringe are connected to a blood vessel of the patient through a bifurcated extension tube,
the cylinder holding mechanism comprises a pair of mechanism for individually holding the cylinder members of the contrast media syringe and the physiological saline syringe,
the cylinder holding mechanism comprises a pair of mechanism for individually sliding the piston members of the contrast media syringe and the physiological saline syringe,
the computer unit individually controls the operations of the pair of piston driving mechanisms,
the main operation panel accepts an input action of various data for normal injection of the contrast media and the physiological saline,
the main display panel displays the various data for normal injection of the contrast media and the physiological saline,
the sub display panel displays various data for test injection of the physiological saline, and
the sub operation panel accepts an input action of the various data for test injection of the physiological saline.

13. A chemical liquid injector according to any one of claims 1 to 11, wherein the liquid syringe includes a contrast media syringe for injecting a contrast media as the liquid into the patient whose image is taken by an imaging diagnostic apparatus and a physiological saline syringe for injecting physiological saline as the liquid into the patient,
the cylinder holding mechanism comprises a pair of mechanism for individually holding the cylinder members of the contrast media syringe and the physiological saline syringe,
the cylinder holding mechanism comprises a pair of mechanism individually sliding the piston members of the contrast media syringe and the physiological saline syringe,
the computer unit individually controls the operations of the pair of piston driving mechanisms,
the main operation panel accepts an input action of various data for normal injection of the contrast media and the physiological saline,
the main display panel displays the various data for normal injection of the contrast media and the physiological saline,
the sub display panel displays various data for test injection of the contrast media, and
the sub operation panel accepts an input action of the various data for test injection of the contrast media.

14. A chemical liquid injector according to claim 13, wherein the contrast media syringe is connected to a blood vessel of the patient through the extension tube,
the computer unit has control data for the piston driving mechanism dedicated to checking of a time period necessary for the contrast media to reach an affected area whose image is taken, the control data being stored as the various data for test injection, and the computer unit controls the operation of the piston driving mechanism in accordance with the control data.

15. A chemical liquid injector according to any one of claims 1 to 14, wherein the main display panel displays a condition screen with its vertical axis representing an injection rate and its horizontal axis representing an injection time of the liquid as the various data for normal injection,
the main operation panel accepts an input action of at least one injection condition comprising an injection rate for each injection time period of the liquid as the various data for normal injection,
the computer unit stores the input injection condition,
the main display panel displays the stored injection condition on the condition screen, and
the computer unit measures at least an elapsed time from the start of injection of the liquid, and controls the operation of the piston driving mechanism in real time in accordance with the elapsed time and the stored injection condition.

16. A chemical liquid injector according to any one of claims 1 to 14, wherein the main display panel displays a condition screen with its vertical axis representing an injection rate and its horizontal axis representing an injection volume of the liquid as the various data for normal injection,
the main operation panel accepts an input action of at least one injection condition comprising an injection rate for each injection volume of the liquid as the various data for normal injection,
the computer unit stores the input injection condition,
the main display panel displays the stored injection condition on the condition screen, and
the computer unit measures at least an injection volume of the liquid from the start of injection, and controls the operation of the piston driving mechanism in real time in accordance with the measured injection volume and the stored injection condition.

17. A chemical liquid injector according to any one of claims 1 to 16, wherein the computer unit stores schematic images of a plurality of body sections of a human body and schematic images of a number of regions to be imaged in relation to each other as the various data for normal injection,
the main display panel displays the schematic images of a plurality of body sections in the shape of a human body,
the main operation panel accepts an input action to select one of the plurality of displayed body sections,
the main display panel displays at least one of the schematic images of the regions to be imaged in association with the selected body section,
the main operation panel accepts an input action to select the displayed region to be imaged, and
the computer unit controls the operation of the piston driving mechanism based on the selected region to be imaged.

18. A chemical liquid injector according to any one of claims 1 to 17, wherein the main operation panel and the main display panel together are a single touch panel for accepting an input action of the various data and displaying output of the data.

19. A chemical liquid injector according to any one of claims 1 to 18, wherein the sub operation panel and the sub display panel together are formed as a single touch panel for accepting an input action of the various data and displaying output of the data.
